# EUROPEAN PATENT SPECIFICATION

(11) **EP 0 424 847 B1**
(45) Date of publication and mention of the grant of the patent: **17.01.1996**
(21) Application number: 90120228.3
(22) Date of filing: 22.10.1990
(51) Int. Cl.: C07C 25/02

(54) **The method for parallel-producing 2,5-dichlorotoluene and 2,6-dichlorotoluene**
Verfahren zur gleichzeitigen Herstellung von 2,5- und 2,6-Dichlortoluol
Procédé pour la préparation simultanée de 2,5- et 2,6-dichlorotoluène

(30) Priority: 23.10.1989 JP 273805/89
(43) Date of publication of application: 02.05.1991
(73) Proprietor: KUREHA CHEMICAL INDUSTRY CO., LTD., Chuo-ku Tokyo 103 (JP)
(72) Inventor: Ishizuka, Makoto, Kawaguchi-shi, Saitama-ken (JP); Hozumi, Toshio, Iwaki-shi, Fukushima-ken (JP)
(74) Representative: Boeters, Hans Dietrich, Dr.

(56) References cited:
- EP-A- 0 257 866
- CHEMICAL ABSTRACTS, vol. 113, no. 1, 2nd July 1990, page 571, abstract no.5899m, Columbus, Ohio, US; & JP-A-02 53 743
- CHEMICAL ABSTRACTS, vol. 107, no. 3, July 1987, page 584, abstract no. 23066t, Columbus, Ohio, US; & JP A-62 05 930
- CHEMICAL ABSTRACTS, vol. 104, no. 7, February 1986, page 496, abstract no. 50631t, Columbus, Ohio, US; & JP-A-60 152 429
- CHEMICAL ABSTRACTS, vol. 105, no. 13, September 1986, page 642, abstract no. 114708d, Columbus, Ohio, US; & JP-A-61 36 234

## Description

The present invention relates to the method for parallel-producing 2,5-dichlorotoluene ( hereinafter referred to as "2,5-DCT") and 2,6-dichlorotoluene (hereinafter referred to as "2,6-DCT").

The 2,5 - DCT and 2,6 - DCT obtained by the method of the present invention are useful as the raw materials for the synthesis of medicines and drugs, agricultural chemicals and other organic compounds.

### Description of the Related Art

When toluene and o-chlorotoluene are just chlorinated in an atempt to make 2,5 - DCT and 2,6 - DCT, there are produced various isomers of dichlorotoluene, and 2,5 - DCT and 2,6 - DCT are obtained merely in a lower yield within their respective isomeric mixtures.

Therefore, the following methods have been proposed for selectively obtaining either 2,5 - DCT alone or 2,6 - DCT alone.
(a) The method for producing 2,5 - DCT through the chlorination and transalkylation of 4-tert-butyltoluene,with Lewis acid and simple substance sulphur as catalysts, European Patent Publication No. 0257866 (Japanese Laid-open Patent Publication No. 63-39828).
(b) 4-tert-butyltoluene is further tert-butylated to 3,5-di-tert-butyltoluene.

The method for producing 2,6 - DCT through the chlorination and transalkylation of the same 3,5-di-tert-butyltoluene with Lewis acid and sulphur compound as catalysts (Japanese Laid-open Patent Publication No. 62-5930).

Both of these methods are for producing 2,5 - DCT or 2,6-DCT with 4-tert-butyltoluene as a raw material. These methods are separately carried out, using separate apparatuses. Namely, the methods are not for parallel-producing 2,5 - DCT and 2,6 - DCT.

As mentioned above, the methods for producing 2,5 - DCT and 2,6 - DCT are respectively known, but accompanied by many by-products. When producing both 2,5 - DCT and 2,6 - DCT, the same raw material, namely 4-tert-butyltoluene is used. Despite this fact, the separate apparatuses are respectively required.

EP-A-0 257 866 describes a process according to which 4-t-butyl toluene is reacted with chlorine in the presence of a Lewis acid (FeCl₃) and sulphur and the resulting 2,5-dichloro-4-t-butyl toluene is contacted with toluene for the production of 2,5-dichloro toluene. Further, JP-A-62.005 930 [Chem. Abstr., 107 (1987) 584 quotation 23 066 t] suggests a reaction of 3,5-di-t-butyl toluene with sulfuryl chloride or chlorine in the presence of a Lewis acid such as AlCl₃ and a sulfur compound such as sulfur monochloride and transalkylation of the resulting 3,5-di-t-butyl-2,6-di-chloro toluene with toluene to 2,6-dichloro toluene. There has, however, still been a need to improve the yields of the chlorinated toluenes and to use mixtures of the starting materials of the prior art.

### Outline of Invention

The object of the present invention is to provide for the method for parallel-producing 2,5 - DCT and 2,6 - DCT with 4-tert-butyl (or isopropyl) toluene as the raw material, using the same chlorination apparatus.

Further, the object of the present invention is to provide for the method for producing 2,5 - DCT and 2,6 - DCT in a higher yield, bringing about a decrease in the formation of by-products.

Further, the object of the present invention is to provide for the method for parallel-producing 2,5 - DCT and 2,6 - DCT at the optional ratio, by adjusting the ratio of raw material 4-tert-butyl (or isopropyl) toluene to raw material 3,5 - di - tert - butyl (or isopropyl) toluene, and using the same chlorination apparatus.

Still further, the object of the present invention is to provide for the method for producing 2,5 - DCT and 2,6 - DCT efficiently, by recyclically using the 4-tert-butyl (or isopropyl) toluene produced in the form of by-products in the production process.

The object of the present invention is attained by providing a method for producing 4-tert-butyl (or isopropyl) -2,5-dichlorotoluene or 3,5-di-tert-butyl (or isopropyl) -2,6-dichlorotoluene, wherein said method is characterized by
(i) chlorinating a mixture of 4-tert-butyl (or isopropyl) toluene and 3,5-di-tert-butyl (or isopropyl) toluene with chlorine in the presence of a Lewis acid and simple substance sulphur, to form 4-tert-butyl (or isopropyl) -2,5-dichlorotoluene and 3,5-di-tert-butyl (or isopropyl) -2,6-dichlorotoluene, and
(ii) causing 4-tert-butyl (or isopropyl) -2,5-dichlorotoluene and 3,5-di-tert-butyl (or isopropyl) -2,6-dichlorotoluene to be separated from the reaction mixture,
The said method according to the invention can be further characterized by
(iii) causing the separated 4-tert-butyl (or isopropyl) -2,5-dichlorotoluene and 3,5-di-tert-butyl (or isopropyl) -2,6-dichlorotoluene to individually contact with twice the amount of moles or more moles of toluene to form 2,5-dichlorotoluene from 4-tert-butyl (or isopropyl) -2,5-dichlorotoluene, and 2,6-dichlorotoluene from 3,5-di-tert-butyl (or isopropyl) -2,6-dichlorotoluene with 4-tert-butyl (or isopropyl) toluene in form of a by-product, respectively, by transalkylation, and isolating same.

Further embodiments of the invention can be drawn from subclaims 3 to 8.

Further, prior to the chlorination process in (i) above, the alkylation process is carried out , involving a partial alkylation of 4-tert-butyl (or isopropyl) toluene in the presence of Lewis acid, for obtaining the mixture of 4-tert-butyl (or isopropyl) toluene and 3,5 - di - tert - butyl (or isopropyl) toluene. By using this mixture as the raw material, the aforementioned processes (i) to (iii), inclusive, are carried out. The 4-tert-butyl (or isopropyl) toluene is produced in the form of by-products in the transalkylation process in (iii). This 4-tert-butyl (or isopropyl) toluene is recyclicaly used as 4-tert-butyl (or isopropyl) toluene or the starting material in the aforementioned alkylation process. Thus, 2,5 - DCT and 2,6 - DCT are obtainable in a high yield.

It is known that ,when 4-tert-butyl (or isopropyl) toluene is chlorinated with Lewis acid and simple substance sulphur as catalysts, 2,5 - dichloro substance is obtained in a high yield. But Lewis acid and sulphur compound have been conventionally used as catalysts for chlorination of 3,5 - di - tert - butyl (or isopropyl) toluene in the process of producing 2,6 - DCT.

However, according to the study made by the present inventors, if the Lewis acid and simple substance sulphur are likewise used as catalysts for the chlorination of 3,5 - di - tert - butyltoluene, 2,6 - dichloro subtance is obtained in far better yield, than the conventional single use of Lewis acid as catalysts or the conventional combined use of Lewis acid and sulphur compound as catalysts. Further, the present inventors have obtained the knowledge that , when Lewis acid and simple substance sulphur are used as chlorination catalysts, there is little difference in the formation rate of dichloro substance of 4-tert-butyl toluene and 3,5 - di - tert - butyltoluene respectively.

Even if they are used in mixed state, their respectively intended dichloro substance may be obtained equally in a high yield and that it be readily separated. The same inventors have also discovered that the 3,5 - di - tert - butyl toluene produced in the form of by-products at the time of producing 4-tert-butyl toluene may be effectively used, and 4-tert-butyl toluene produced at the time of producing 3,5 - di - tert - butyl toluene be likewise used.

According to the present invention, therefore, 2,5 - DCT and 2,6 - DCT may be parallel-produced at the optional ratio, by adjusting the ratio of 4-tert-butyl toluene and 3,5 - di - tert - butyl toluene and using one and the same chlorination equipment.

In addition, the aforementioned word "4-tert-butyl (or isopropyl) toluene " signifies 4-tert- butyl toluene or 4-isopropyltoluene. The words "3,5 - di - tert - butyl (or isopropyl) toluene", "4 - tert -butyl (or isopropyl) - 2,5 - dichlorotoluene" and "3,5 - di - tert - butyl (or isopropyl)-2,6 - dichlorotoluene" are used similarly.

Then, reference is hereinafter made to the means for the present invention.

Both 2,5 - DCT and 2,6 - DCT involve the 4-tert-butyl (or isopropyl) toluene as the raw material. They are produced in a similar process either when the raw material is 4-tert-butyl toluene or when it is 4-isopropyl toluene. Therefore, full particulars are given, as mentioned below, about the case where 4-tert-butyl toluene (hereinafter referred to as "BT") is used as the raw material.

### 1) Alkylation Process

The alkylation process is the process in which there is alkylated a part of raw material BT and obtained the mixture of BT with 3,5-di-tert-butyltoluene (hereinafter referred to "DBT") at the desired ratio. The mixture obtained thereby is used as the raw material for chlorinaton.

The degree of tert-butylation of BT is determined by the production ratio of intended 2,5 - DCT and 2,6 - DCT . Those used as tert-butylation agents are tert-butylchloride and isobutylene (be that as it may, those used as alkylation agents for 4-isoproplytoluene are isopropylchloride and propylene). The tert-butylation involving the use of tert-butylchloride as tert-butylation agent is carried out, by using the reactor provided with a stirrer, dropping apparatus, reflux condenser and thermometer, drawing thereinto BT as raw material and Lewis acid as catalyst , and adding dropwise tert-butylchloride for reaction.

Those used as Lewis acids may be , for example, AlCl₃ , FeCl₃, etc. The amount thereof used is preferably in an amount corresponding to 1 - 15 mole % per raw material BT. The reaction temperature is 10 - 80°C , preferably 15 - 25 °C. If the reaction temperature is too low, the reaction rate would become slower. On the contrary, if it is too high, by-products would increase and DBT slectivity be lowered. The ratio of BT and DBT in the reaction product is determined by the molar ratio of BT and tert-butylchloride.

Further, BT is sequentially fed, in the alkylation process, in an amount proportionate to the total of the amount of 2,5 - DCT and 2,6 - DCT produced and the amount lost throughout the process. This new BT is manufactured through the reaction of toluene with tert-butylchloride or isobutylene. At this time, however, DBT is inevitably produced besides BT. Therefore, when 2,5 - DCT alone is intended to be produced, a separation is required between BT and DBT. The DBT portion results in a loss. When intending to produce 2,6 - DCT alone, BT is likewise required to be separated. When, however, 2,5 - DCT and 2,6 - DCT are parallel-produced as in the case of the present invention, the above loss would be avoided.

### (2) Chlorination process

When there is added to the mixture of BT and DBT obtained in the alkylation process the DBT obtained in the subsequent process, and subjected to chlorination, there is produced, from BT, 4 - tert -butyl - 2,5 - dichlorotoluene ( hereinafter referred to as " B-2,5 -DCT" ), and , from DBT, 3,5 - di - tert - butyl-2,6 - dichlorotoluene (hereinafter referred to as "DBDCT").

The present invention is based on the discovery that, when Lewis acid and simple substance sulphur are used as chlorination catalysts, DBDCT improves in its yield and difference is hardly observed between the chlorination of BT rate and that of DBT. When the mixture of BT and DBT is chlorinated up to 1.9 - 2.0 of chlorination degree, dichloro substance is obtained in such high a yield as when BT and DBT are independently chlorinated.

Further, the term "chlorination degree" as used herein denotes an average number of chlorine atom or chlorine atoms combined to a benzene ring.

The above chlorination may be carried out by adding, to the BT alkylated liquid to which the DBT produced in the subsequent process is added, or the mixture of BT and DBT containing alkylation catalyst, simple substance sulphur as a promoter and introducing there into chlorination agent. Further, the existence of simple substance sulphur contributes to higher selectivity between BT and B-2,5 - DCT. Not only that, but it improves the yield of DBDCT from DBT (Japanese Patent Application No. 63-203238). The amount of simple substance sulphur used in 50 - 150 mole % , preferably 80 - 120, mole % of Lewis acid used at the time of alkylation of BT.

Lewis acid and simple substance sulphur may be added in the chlorination process. Also, Lewis acid may be added in the alkylation process and be subjected to alkylation, and then simple substance sulphur be added in the chlorination process. Such thing is called "in the presence of Lewis acid and simple substance sulphur" under the present invention.

In addition, the aforementioned chlorination may be carried out in the presence of solvent. The examples of solvents used are carbon tetrachloride, chloroform, nitromethane, nitropropane, nitrobenzene, etc. The amount of solvent used is the amount sufficient to maintain the concentration of total BT and DBT at 50 or more % by weight. In addition, the reaction temperatures are set in the range of -10 ∼ 80°C , preferably 10 ∼ 60 °C. If the reaction temperature is too low, the reaction rate would become slower. This is not practical. On the contrary, if it is too high, by-products would increase and the slectivity of B-2,5 - DCT and DBDCT or end-products be lowered. Therefore, the above range is preferable.

### (3) Separation process

The liquid mixture obtained in the chlorination process is subjected to distillation and crystallization. Thus, B-2,5 - DCT and DBDCT are separated.

### (4) Process for obtaining 2,5 - DCT and 2,6 - DCT

The B-2,5 - DCT and DBDCT separated in the separation process are individually caused to react with toluene , in the presence of Lewis acid, to obtain 2,5 - DCT and BT, and 2,6 - DCT and BT. The amount of toluene used at this time is twice or more moles, preferably 10 - 25 times by mole, as much as that of B-2,5 - DCT or DBDCT. The reaction is carried out in the presence of Lewis acid at 10 - 60 °C for 4 - 5 hours.

In addition, when 2,6 - DCT is separated by distillation from transalkylated liquid of DBDCT and toluene, disproportionation occurs and DBT is formed. This DBT is served for chlorination together with the alkylated liquid of BT. BT may be further served for disproportionation.

The present invention relates to the method for parallel-producing 2,5 - DCT and 2,6 - DCT. No separation is required to be made between the BT and DBT obtained by partially alkylating BT. The mixture per se is subjected to chlorination. Therefore, when producing 2,5 - DCT and 2,6 - DCT separately, there is such advantage that the impurity as the chlorination raw material may be effectively used without being separated from BT for manufacturing 2,5 - DCT or from DBT for manufacturing 2,6 - DCT.

Further, according to the present invention, one chlorination equipment is sufficient, despite the fact that the production of two kinds of chlorinated products are involved. The separation of the chlorinated products obtained is also easy. Further, due to the combined use of Lewis acid and simple substance sulphur, the yield of 2,6 - DCT improves, compared with the conventional method, and by-products are decreased. Still further, there is such advantage in industry that the ratio of 2,5 - DCT with 2,6 - DCT may be optionally changed.

### (5) Detailed description of embodiments of invention

The present invention is illustrated, as mentioned below, by way of examples. It should, however, be borne in mind that the present invention is not limited to the following examples only.

### Example 1

### (1) (Alkylation of BT)

Into a 5 liter six neck flask provided with a stirrer, dropping apparatus, reflux condenser and thermometer are introduced 937.8 g of BT (6.34 mol) and 42.3 g of AlCl₃(0.317 mol). Thereto is dropwise added 586.1g of tert-butylchloride(hereinafter referred to as "t-BuC) by a dropping apparatus in two hours. During this time, the reaction temperature is maintained at 15 ∼ 20 °C and further it was continuously stirred for five hours. After the reaction was over, 100 m*l* of N₂ gas per minute was blown into the reaction mixture for two hours for removing the unreacted t-BuC remaining therein.

From the volume of liquid reaction mixture obtained and the analysis of composition thereof by gas chromatography, the yields of BT and DBT were found to be 141.7g and 1097.3g.

### (2) (Chlorination of BT and DBT)

After the alkylation of BT was over, 752.7g of DBT produced in the subsequent process and 10.1g of simple substance sulphur (0.317 mol) were added there to and continuously stirred, the temperature being maintained at 20°C.

At this temperature, it was chlorinated at the rate of 10 mol of chlorine per hour for 1 ¹/₄ hours.

Then, the reaction mixture was sequentially washed with hydrochloric acid (HCl content, 20 % by weight), 10 wt % aqueous solution of sodium carbonate, and water. It was finally dried with anhydrous sodium sulfate. This product contained, as the main ingredient, 149.6 g of B-2,5-DCT(yield, 72%) and 1807.4 g of DBDCT (yield, 73.0 %). This mixture was subjected to rectification treatment by a distillation tower (number of steps, 50).

There were obtained the fraction of 114°C /667 Pa (5 Torr):143.2g (B-2,5-DCT: 142.1g) and the fraction of 130°C /667 Pa (5 Torr):1730.8 g (DBDCT: 1717g). Their distillation yields were respectively 95%.

### (3) (Transalkylation of B-2,5-DCT and DBDCT)

### ① Transalkylation of B-2,5-DCT

Into a 2 liter four neck flask provided with a stirrer, condenser and thermometer are introduced 143.2 g of crude B-2,5-DCT(B-2,5-DCT : 142.1g: 0.65 mol) obtained as mentioned in (2) above , 8.68 g of AlCl₃(0.065 mol) and 1196 g (13 mol) of toluene. It was subjected to reaction at 30 °C for five hours.

From the volume of liquid reaction mixture obtained and the analysis of composition thereof by gas chromatography, the output (yield) of BT and 2,5-DCT was found to be respectively 96.0g (yield, 99%) and 104.4g (yield, 99%). After the reaction mixture was washed with 5 wt % aqueous solution of sodium carbonate, toluene was removed. The mixture of BT and 2,5-DCT was subjected to rectification treatment by a distillation tower (number of steps, 50). There were obtained the fraction of 57°C /667 Pa (5Torr):96.9g (BT: 95.9g) and the fraction of 62 - 63°C /667 Pa (5Torr):100.0g (2,5-DCT: 99.2g,)

### ② Transalkylation of DBDCT

Into a 20 liter four neck flask provided with a stirrer, condenser and thermometer are introduced 1730.8 g of crude DBDCT(DBDCT 1717g: 6.29 mol) obtained as mentioned in (2) above , 84.0 g of AlCl₃(0.629 mol) and 11600 g of toluene (126 mol). It was subjected to reaction at 30 °C for five hours.

From the volume of liquid reaction mixture obtained and the analysis of composition thereof by gas chromatography, the output (yield) of BT and 2,6-DCT was found to be respectively 1768.6g (yield, 95%) and 941.7g (yield, 93%). Then, the reaction mixture per se is introduced into a distillation tower (number of steps, 50).

Under 4 000 - 8 000 Pa (30 - 60 Torr) and 60 °C of liquid temperature in the bottom of the tower, toluene was removed. This condition was maintained for 12 hours. After the catalyst was removed, the rectification treatment was repeated.

There were obtained the fraction of 57°C /667 Pa (5 Torr):594g (BT: 588g) , the fraction of 63°C /667 Pa (5 Torr):900.0g (2,6-DCT: 8946g) and the fraction of 90 - 92°C /667 Pa (5 Torr): 758.8g(DBT: 752.7g). In addition, 758.8g of crude DBT obtained in this example (DBT:752.7g) may be returned to the chlorination process of BT and DBT.

Further, reference is made to the amount of BT which may be returned to the alkylation process of BT. The amount of BT recovered would be 633.9g through the combination of the aforementioned 588 g and 95.9 g recovered in the transalkylation process of Example 1, (3) ①.

### Example 2

### (1) (Alkylation of BT)

Into a 5 liter six neck flask provided with a stirrer, dropping apparatus, reflux condenser and thermometer are introduced 1237g of BT (8.358 mol) and 44.6 g of AlCl₃(0.334 mol). There to is dropwise added 386.6g of t-BuC(4.179 mol) by a dropping apparatus in two hours.

During this time, the reaction temperature is maintained at 15 - 20 °C and further it was continuously stirred for three hours.

After the reaction was over, 50 m*l* of N₂ gas per minute was blown into the reaction mixture for one hour for removing the unreacted t-BuC remaining therein.

From the volume of liquid reaction mixture obtained and the analysis of composition thereof by gas chromatography, the yields of BT and DBT were found to be 708.7 and 728.4 g.

### (2) (Chlorination of BT and DBT)

After the alkylation of BT was over, 305g of DBT produced in the subsequent process and 10.7g of simple substance S (0.334 mol) were added thereto and continuously stirred, the temperature being maintained at 20°C.

At this temperature, it was chlorinated at the rate of 10.0 mol of chlorine per hour for 1 ¹/₄ hours.

Then, the reaction mixture was sequentially washed with hydrochloric acid (HCl content,20 % by weight), 10 wt% aqueous solution of sodium carbonate, and water. It was finally dried with anhydrous sodium sulfate.

This product contained, as the main ingredient, 748.2 g of B-2,5-DCT(yield, 72%) and 959 g of DBDCT (yield, 73.0 %).

This mixture was subjected to rectification treatment by a distillation tower (number of steps, 50).

There were obtained the fraction of 114°C /667 Pa (5 Torr):716.5 g (B-2,5-DCT: 710.8g) and the fraction of 130°C /667 Pa (5 Torr):966.7g (DBDCT: 959g).

Their distillation yields were respectively 95%.

### (3) (Transalkylation of B-2,5-DCT and DBDCT)

### ① Transalkylation of B-2,5-DCT

Into a 10 liter four neck flask provided with a stirrer, condenser and thermometer are introduced 716.5g of crude B-2,5-DCT(B-2,5-DCT :710.8g: 3.276 mol) obtained as mentioned in (2) above , 43.73g of AlCl₃(0.327 mol) and 6030 g of toluene (65.54 mol). It was subjected to reaction at 30 °C for five hours.

From the volume of liquid reaction mixture obtained and the analysis of composition thereof by gas chromatography, the output (yield) of BT and 2,5-DCT was found to be respectively 479.9g (yield, 99%) and 522.1g (yield, 99%).

After the reaction mixture was washed with 5 wt % aqueous solution of sodium carbonate, toluene was removed. The mixture of BT and 2,5-DCT was subjected to rectification treatment by a distillation tower (number of steps, 50).

There were obtained the fraction of 57°C /667 Pa (5 Torr):458.6g (BT: 454.0g) and the fraction of 62 - 63°C /667 Pa (5 Torr):500.0g (2,5-DCT: 496g).

### ② Transalkylation of DBDCT

Into a 10 liter four neck flask provided with a stirrer, condenser and thermometer are introduced 966.7 g of crude DBDCT(DBDCT 959g:3.513 mol) obtained as mentioned in (2) above , 46.9 g of AlCl₃(0.3513 mol) and 6463.4g of toluene (70.26 mol). It was subjected to reaction at 30 °C for five hours.

From the volume of liquid reaction mixture obtained and the analysis of composition thereof by gas chromatography, the output (yield) of BT and 2,6-DCT was found to be respectively 987.8g (yield, 95%) and 526g (yield, 93%).

Then, the reaction mixture per se is introduced into a distillation tower (number of steps, 50).

Under 4 000 - 8 000 Pa (30 - 60 Torr) and 60 °C of liquid temperature in the bottom of the tower, toluene was removed. This condition was maintained for 12 hours. After the catalyst was removed, the rectification treatment was repeated.

There were obtained the fraction of 57°C /667 Pa (5 Torr):331.8g (BT: 328.5g), the fraction of 63°C /667 Pa (5 Torr):500.0g (2,6-DCT: 497g) and the fraction of 90 - 92°C /667 Pa (5 Torr):307.4g (DBT: 305g). In addition, 307.4g of crude DBT obtained in this example (DBT: 305g) may be returned to the chlorination process of BT and DBT.

Further, reference is made to the amount of BT which may be returned to the alkylation process of BT. The amount of BT recovered would be 782.5g through the combination of the aforementioned 328.5 g and 454.0 g recovered in the transalkylation process of Example 2, (3) ①.

### Example 3

### (1) (Alkylation of BT)

Into a 5 liter six neck flask provided with a stirrer, dropping apparatus, reflux condenser and thermometer are introduced 1397.1g of BT (9.44 mol) and 50.4 g of AlCl₃(0.378 mol). Thereto is dropwise added 87.32g of t-BuC(0.944 mol) by a dropping apparatus in one hour.

During this time, the reaction temperature is maintained at 15 - 20 °C and further it was continuously stirred for five hours.

After the reaction was over, 50 m*l* of N₂ gas per minute was blown into the reaction mixture for one hour for removing the unreacted t-BuC remaining therein.

From the volume of liquid reaction mixture obtained and the analysis of composition thereof by gas chromatography, the yields of BT and DBT were found to be 1275.8 and 167.2g.

### (2) (Chlorination of BT and DBT)

After the alkylation of BT was over, 38.2g of DBT produced in the subsequent process and 12.10g of simple substance sulphur (0.378 mol) were added thereto and continuously stirred, the temperature being maintained at 20°C.

At this temperature, it was chlorinated at the rate of 10.0 mol of chlorine per hour for 1 ¹/₄ hours.

Then, the reaction mixture was sequentially washed with hydrochloric acid (HCl content, 20 % by weight), 10 wt% aqueous solution of sodium carbonate, and water. It was finally dried with anhydrous sodium sulfate.

This product contained, as the main ingredient, 1346.8g of B-2,5-DCT(yield, 72%) and 200.7g of DBDCT (yield, 73.0 %).

This mixture was subjected to rectification treatment by a distillation tower (number of steps, 50).

There were obtained the fraction of 114°C /667 Pa (5 Torr):1289.8g (B-2,5-DCT: 1279.5g) and the fraction of 130°C /667 Pa (5 Torr):192.2g (DBDCT: 190.7g).

Their distillation yields were respectively 95%.

### (3) (Transaklylation of B-2,5-DCT and DBDCT)

### ① Transaklyation of B-2,5-DCT

Into a 20 liter four neck flask provided with a stirrer, condenser and thermometer are introduced 1289.8g of crude B-2,5-DCT(B-2,5-DCT : 1279.5g: 5.896 mol) obtained as mentioned in (2) above , 78.8g of AlCl₃(0.59 mol) and 10856 g(118 mol) of toluene. It was subjected to reaction at 30 °C for five hours.

From the volume of liquid reaction mixture obtained and the analysis of composition thereof by gas chromatography, the output (yield) of BT and 2,5-DCT was respectively 863.9g (yield, 99%) and 939.8g (yield, 99%).

After the reaction mixture was washed with 5 wt % aqueous solution of sodium carbonate, toluene was removed. The mixture of BT and 2,5-DCT was subjected to rectification treatment by a distillation tower (number of steps, 50).

There were obtained the fraction of 57°C /667 Pa (5 Torr):829g (BT: 820.7g) and the fraction of 62 - 63°C /667 Pa (5 Torr):900.0g (2,5-DCT: 892.8g).

### ② Transalkylation of DBDCT

Into a 3 liter four neck flask provided with a stirrer, condenser and thermometer are introduced 192.2 g of crude DBDCT(DBDCT 190.7g:0.699 mol) obtained as mentioned in (2) above , 9.35g of AlCl₃(0.07 mol) and 1288g of toluene (14 mol). It was subjected to reaction at 30 °C for five hours.

From the volume of liquid reaction mixture obtained and the analysis of composition thereof by gas chromatography, the output (yield) of BT and 2,6-DCT was found to be respectively 123.6g (yield, 95%) and 104.6g (yield, 93%).

Then, the reaction mixture per se is introduced into a distillation tower (number of steps, 50).

Under 4 000 - 8 000 Pa (30 - 60 Torr) and 60 °C of liquid temperature in the bottom of the tower, toluene was removed. This condition was maintained for 6 hours. After the catalyst was removed, the rectification treatment was repeated.

There were obtained the fraction of 57°C /667 Pa (5 Torr):41.5g (BT:41.0g), the fraction of 63°C /667 Pa (5 Torr):100.0g (2,6-DCT: 99.4g) and the fraction of 90 - 92°C /667 Pa (5 Torr):38.5g (DBT: 38.2g).

In addition, 38.5g of crude DBT obtained in this example (DBT: 38.2g) may be returned to the chlorination process of BT and DBT.

Further, reference is made to the amount of BT which may be returned to the alkylation process of BT. The amount of BT recovered would be 861.7g through the combination of the aforementioned 41.0g and 827.7g recovered in the transalkylation process of Example 3, (3) ①.

### Referential example

This referential example denotes difference in yield, depending upon the catalyst employed.

Into a 300 ml separable flask provided with a stirrer, reflux condenser and thermometer are respectively introduced, in a dark room, 81.6 g(0.4 mol) of DBT, 25 ml of carbon tetrachloride as solvent and catalysts described in the following table. It was maintained at 20°C. This mixture was subjected to 0.8 mol of chlorine gas for one hour and caused to react. Hydrochloric acid gas generated was introduced into an aqueous solution of sodium hydroxide.

Then, the reaction mixture was washed with water. After the organic phase thereof was dried with anhydrous sodium sulfate, the solvent was removed by distillation.

The following table shows the yields of DBDCT obtained in view of the analysis of reaction mixture composition by gas chromatography. From this, it is understood that the yield of DBDCT is high when Lewis acid and simple substance sulphur are used as catalysts.

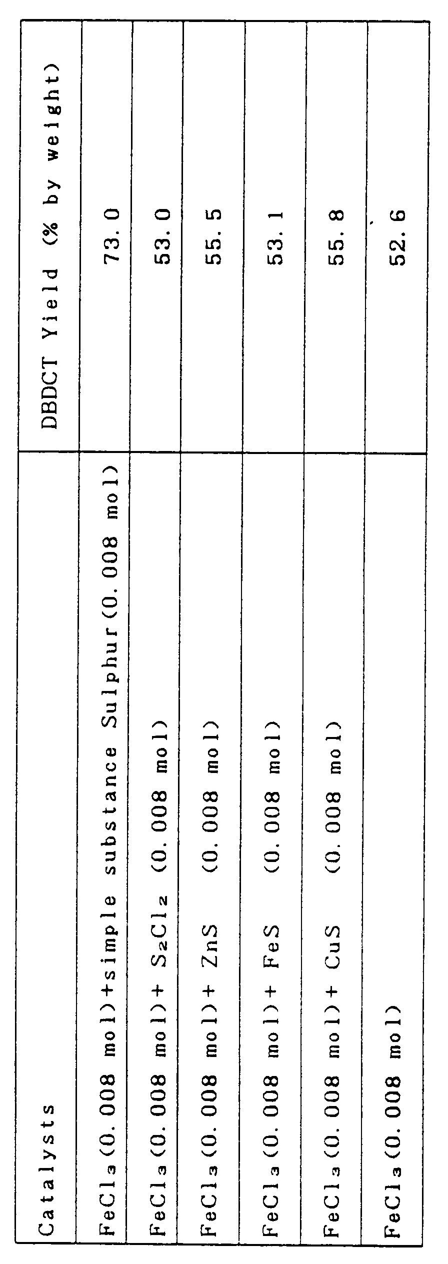

## Claims

1. A method for producing 4-tert-butyl (or isopropyl) -2,5-dichlorotoluene or 3,5-di-tert-butyl (or isopropyl) -2,6-dichlorotoluene, characterized by
(i) chlorinating a mixture of 4-tert-butyl (or isopropyl) toluene and 3,5-di-tert-butyl (or isopropyl) toluene with chlorine in the presence of a Lewis acid and simple substance sulphur, to form 4-tert-butyl (or isopropyl) -2,5-dichlorotoluene and 3,5-di-tert-butyl (or isopropyl) -2,6-dichlorotoluene, and
(ii) causing 4-tert-butyl (or isopropyl) -2,5-dichlorotoluene and 3,5-di-tert-butyl (or isopropyl) -2,6-dichlorotoluene to be separated from the reaction mixture.

2. A method according to claim 1, further characterized by
(iii) causing the separated 4-tert-butyl (or isopropyl) -2,5-dichlorotoluene and 3,5-di-tert-butyl (or isopropyl) -2,6-dichlorotoluene to individually contact with twice the amount of moles or more moles of toluene to form 2,5-dichlorotoluene from 4-tert-butyl (or isopropyl) -2,5-dichlorotoluene, and 2,6-dichlorotoluene from 3,5-di-tert-butyl (or isopropyl) -2,6-dichlorotoluene with 4-tert-butyl (or isopropyl) toluene in form of a by-product, respectively, by transalkylation, and isolating same.

3. The method of claim 1, wherein there is used as the raw material a mixture of 4-tert-butyl (or isopropyl) toluene and 3,5-di-tert-butyl (or isopropyl) toluene obtained by partially alkylating 4-tert-butyl (or isopropyl) toluene in the presence of a Lewis acid.

4. The method of claim 3, wherein their is cyclically used 4-tert-butyl (or isopropyl) toluene produced in the form of a by-product, as raw material for the alkylation process.

5. The method according to claim 4, wherein there is used 4-tert-butyl (or isopropyl) toluene containing a Lewis acid.

6. The method according to any of claims 1 to 5, wherein chlorination is carried out at a chlorination degree of 1.9 to 2.0.

7. The method according to any of claims 1 to 6, wherein the amount of simple substance sulphur used corresponds to 50 to 150 mol % of the Lewis acid.

8. The method according to any of claims 1 to 7, wherein chlorination is carried out at a temperature of from -10 to 80 °C.

## Patentansprüche

1. Verfahren zur Herstellung von 4-tert-Butyl-(oder Isopropyl-)-2,5-dichlortoluol oder 3,5-Di-tert-Butyl-(oder Isopropyl-)-2,6-dichlortoluol, dadurch gekennzeichnet,
i) daß man eine Mischung aus 4-tert-Butyl-(oder Isopropyl-)-toluol und 3,5-Di-tert-Butyl-(oder Isopropyl-)-toluol mit Chlor in Gegenwart einer Lewis-Säure und elementarem Schwefel unter Bildung von 4-tert-Butyl-(oder Isopropyl-)-2,5-dichlortoluol und 3,5-Di-tert-Butyl-(oder Isopropyl-)-2,6-dichlortoluol chloriert und
ii) 4-tert-Butyl-(oder Isopropyl-)-2,5-dichlortoluol und 3,5-Di-tert-Butyl-(oder Isopropyl-)-2,6-dichlortoluol vom Reaktionsgemisch abtrennt.

2. Verfahren nach Anspruch 1, zusätzlich gekennzeichnet durch
iii) getrenntes Inberührungbringen des separierten 4-tert-Butyl-(oder Isopropyl-)-2,5-dichlortoluol und 3,5-Di-tert-Butyl-(oder Isopropyl-)-2,6-dichlortoluol mit der zweifachen Menge an Mol oder einer größeren Menge Mol von Toluol und Bildung von 2,5-Dichlortoluol aus 4-tert-Butyl-(oder Isopropyl-)-2,5-dichlortoluol und 2,6-Dichlortoluol aus 3,5-Di-tert-Butyl-(oder Isopropyl-)-2,6-dichlortoluol mit 4-tert-Butyl-(oder Isopropyl-)-toluol in Form eines Nebenprodukts durch Transalkylieren und Isolieren.

3. Verfahren nach Anspruch 1, bei dem man als Rohmaterial eine Mischung aus 4-tert-Butyl-(oder Isopropyl-)-toluol und 3,5-Di-tert-Butyl-(oder Isopropyl-)-toluol verwendet, erhalten durch partielles Alkylieren von 4-tert-Butyl-(oder Isopropyl)-toluol in Gegenwart einer Lewis-Säure.

4. Verfahren nach Anspruch 3, bei dem man cyclisch 4-tert-Butyl-(oder Isopropyl-)-toluol, gebildet in Form eines Nebenprodukts, als Ausgangsmaterial für den Alkylierungsprozeß verwendet.

5. Verfahren nach Anspruch 4, bei dem man 4-tert-Butyl-(oder Isopropyl-)-toluol mit einem Gehalt an einer Lewis-Säure verwendet.

6. Verfahren nach einem der Ansprüche 1 bis 5, bei dem man die Chlorierung bei einem Chlorierungsgrad von 1,9 bis 2,0 durchführt.

7. Verfahren nach einem der Ansprüche 1 bis 6, bei dem die Menge an eingesetztem elementaren Schwefel 50 bis 150 mol-% der Lewis-Säure entspricht.

8. Verfahren nach einem der Ansprüche 1 bis 7, bei dem man die Chlorierung bei einer Temperatur von -10 bis 80 °C durchführt.

## Revendications

1. Un procédé pour la préparation du 4-tert-butyl (ou isopropyl) -2,5-dichlorotoluène ou du 3,5-di-tert-butyl (ou isopropyl)-2,6-dichlorotoluène, caractérisé par
(i) la chloration d'un mélange de 4-tert-butyl (ou isopropyl) toluène et de 3,5-di-tert-butyl (ou isopropyl) toluène avec du chlore en présence d'un acide de Lewis et de fleur de soufre, afin de former du 4-tert-butyl (ou isopropyl)-2,5-dichlorotoluène et du 3,5-di-tert-butyl (ou isopropyl) -2,6-dichlorotoluène, et
(ii) le fait que l'on provoque la séparation, du mélange réactionnel, du 4-tert-butyl (ou isopropyl) -2,5-dichlorotoluène et du 3,5-di-tert-butyl (ou isopropyl)-2,6-dichlorotoluène.

2. Un procédé selon la revendication 1, caractérisé en outre par (iii) le fait que l'on provoque la mise en contact individuel du 4-tert-butyl (ou isopropyl) -2,5-dichlorotoluène et du 3,5-di-tert-butyl (ou isopropyl)-2,6-dichlorotoluène séparés avec deux fois la quantité de moles ou plus de moles de toluène afin de former du 2,5-dichlorotoluène à partir du 4-tert-butyl (ou isopropyl)-2,5-dichlorotoluène, et du 2,6-dichlorotoluène à partir du 3,5-di-tert-butyl (ou isopropyl)-2,6-dichlorotoluène avec du 4-tert-butyl (ou isopropyl) toluène sous la forme d'un produit secondaire, respectivement, par transalkylation, suivi d'un isolement.

3. Le procédé de la revendication 1, dans lequel est utilisé en tant que matière première un mélange de 4-tert-butyl (ou isobutyl) toluène et de 3,5-di-tert-butyl (ou isopropyl) toluène obtenu par alkylation partielle du 4-tert-butyl (ou isopropyl) toluène en présence d'un acide de Lewis.

4. Le procédé de la revendication 3, dans lequel est cycliquement utilisé du 4-tert-butyl (ou isopropyl) toluène préparé sous la forme d'un produit secondaire, en tant que matière première pour la technique d'alkylation.

5. Le procédé selon la revendication 4, dans lequel est utilisé du 4-tert-butyl (ou isopropyl) toluène renfermant un acide de Lewis.

6. Le procédé selon l'une quelconque des revendications 1 à 5, dans lequel la chloration est mise en oeuvre selon un degré de chloration de 1,9 à 2,0.

7. Le procédé selon l'une quelconque des revendications 1 à 6, dans lequel la quantité de fleur de soufre utilisée correspond à 50 à 150 % molaires de l'acide de Lewis.

8. Le procédé selon l'une quelconque des revendications 1 à 7, dans lequel la chloration est mise en oeuvre à une température de -10 à 80°C.
